(19) Europäisches Patentamt
European Patent Office
Office européen des brevets

(11) Veröffentlichungsnummer: **0 099 516**
**A1**

(12) # EUROPÄISCHE PATENTANMELDUNG

(21) Anmeldenummer: **83106607.1**

(51) Int. Cl.³: **C 07 C 85/24**

(22) Anmeldetag: **06.07.83**

(30) Priorität: **17.07.82 DE 3226889**

(43) Veröffentlichungstag der Anmeldung:
**01.02.84 Patentblatt 84/5**

(84) Benannte Vertragsstaaten:
**DE FR GB IT**

(71) Anmelder: **BASF Aktiengesellschaft**
**Carl-Bosch-Strasse 38**
**D-6700 Ludwigshafen(DE)**

(72) Erfinder: **Wolff, Dietrich, Dr.**
**Max-Reger-Strasse 3a**
**D-6831 Plankstadt(DE)**

(72) Erfinder: **Hertel, Otto, Dr.**
**Koenigsbacher Strasse 68**
**D-6700 Ludwigshafen(DE)**

(72) Erfinder: **Voges, Dieter, Dr.**
**Richard-Wagner-Strasse 28**
**D-6800 Mannheim 1(DE)**

(54) **Verfahren zur Herstellung von Bis(aminocyclohexyl)dialkylmethanen.**

(57) Verfahren zur Herstellung von Bis(aminocyclohexyl)dialkylmethanen der allgemeinen Formel I

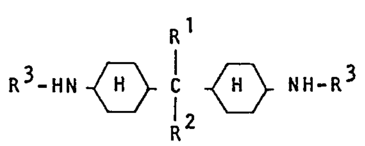

(I)

in der

$R^1$ und $R^2$ gleich oder verschieden sein können und eine niedere Alkylgruppe und

$R^3$ ein Wasserstoffatom oder eine niedere Alkylgruppe bedeuten, wobei die beiden Cyclohexanringe gegebenenfalls substituiert sein können, durch Kondensation von in para-Stellung unsubstituierten Anilinen mit niederen aliphatischen Ketonen, in Gegenwart einer starken Säure und nachfolgende Hydrierung der so erhaltenen Produkte, in Gegenwart von Ruthenium enthaltenden Katalysatoren.

EP 0 099 516 A1

Verfahren zur Herstellung von Bis(aminocyclohexyl)dialkylmethanen

Die vorliegende Erfindung betrifft ein neues Verfahren zur Herstellung von Bis(aminocyclohexyl)dialkylmethanen durch Umsetzung von in para-Stellung unsubstituierten Anilinen mit aliphatischen Ketonen zu Diphenylmethanderivaten und deren anschließende Kernhydrierung.

Es ist bekannt, Anilin oder seine in para-Stellung unsubstituierten Derivate mit Formaldehyd in wäßriger Lösung, in Gegenwart von Mineralsäuren, zu Bis(aminophenyl)-methanverbindungen umzusetzen, vgl. dazu Kirk-Othmer, Encyclopedia of Chemical Technology, 3. Auflage, Band 2, S. 338 f.

Dagegen ist die Kondensation von in para-Stellung unsubstituierten Anilinen mit aliphatischen Ketonen weniger untersucht.

In der DE-PS 399 149 wurde erstmals die Umsetzung von Anilinhydrochlorid mit aliphatischen Ketonen in wäßriger Lösung, in Gegenwart von Salzsäure beschrieben.

Allerdings konnte gezeigt werden (J. Amer. Chem. Soc. 60, 1458(1938)), daß in wäßriger Lösung unter Einfluß relativ hoher Salzsäure - Konzentrationen, bei einer Arbeitstemperatur von 120 bis 150°C bevorzugt Chinolin - bzw. Dihydrochinolinderivate entstehen. Die Bis(aminophenyl)dialkylmethane werden unter diesen Bedingungen nur als Nebenprodukte gebildet.

Dabei wirkt sich hinsichtlich der Bildung und Isolierung der gewünschten Produkte die Anwesenheit von gleichzeitig

Kg/P

entstehendem Diphenylamin besonders nachteilig aus, da es mit den Bis(aminophenyl)dialkylmethanen in Gegenwart von Salzsäure unerwünschte Folgeprodukte liefert.

Der zweite Reaktionsschritt, die Hydrierung der aromatischen Ringe mittels Rutheniumkatalysatoren bzw. Kobalt-, Nickel, Platin- oder Palladiumkatalysatoren ist in der DE-PS 1 282 018 bzw. in der dort zitierten Literatur beschrieben. Wie aber aus den Ausführungsbeispielen dieser Schrift hervorgeht, werden dabei Desaminierungs- und Kondensationsprodukte gebildet, die das Endprodukt verunreinigen und seine Ausbeute erniedrigen.

Auch die Verwendung von Ruthenium-Nickel-Katalysatoren liefert beträchtliche Mengen an Zersetzungs- und höhersiedenden Kondensationsprodukten (US-PS 2 606 925).

Es wurde nun gefunden, daß man Bis(aminocyclohexyl)-dialkylmethane der allgemeinen Formel I

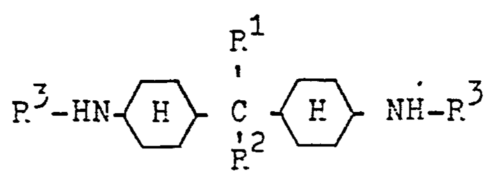 (I)

in der
$R^1$ und $R^2$ gleich oder verschieden sein können und eine niedere Alkylgruppe mit 1 bis 4 Kohlenstoffatomen und $R^3$ ein Wasserstoffatom oder eine niedere Alkylgruppe mit 1 bis 4 Kohlenstoffatomen bedeuten, wobei die beiden Cyclohexanringe gegebenenfalls jeweils durch eine oder zwei niedere Alkylgruppen, mit 1 bis 4 Kohlenstoffatomen oder durch eine Carboxyalkylgruppe mit 1 bis 4 Kohlenstoffatomen im Alkylrest substituiert sein können, durch Kondensation von in para-Stellung unsubstituierten

Anilinen der allgemeinen Formel II

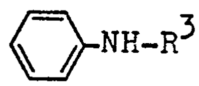 (II)

in der $R^3$ die vorgenannte Bedeutung besitzt und die gegebenenfalls am Kern durch eine oder zwei niedere Alkylgruppen, mit 1 bis 4 Kohlenstoffatomen oder durch eine Carboxyalkylgruppe mit 1 bis 4 Kohlenstoffatomen im Alkylrest substituiert sein können, mit niederen aliphatischen Ketonen, deren Alkylreste jeweils 1 bis 4 Kohlenstoffatome aufweisen, in Gegenwart einer starken ein- oder mehrbasigen Säure und nachfolgende Hydrierung der so erhaltenen Produkte, vorteilhaft herstellen kann, wenn man die Kondensation bei einer Temperatur von 100 bis 160°C und einem Druck von 1 bis 20 bar, in Anwesenheit von 10 bis 60 Gew.-% Wasser, bezogen auf das Reaktionsgemisch, durchführt, wobei das Molverhältnis Anilin:Keton 2:1 bis 10:1 und das Molverhältnis Anilin:Säureäquivalent 1:1 bis 4:1 beträgt, und die nachfolgende Hydrierung der so erhaltenen Produkte bei einer Temperatur von 30 bis 250°C und einem Druck, der größer als 50 bar ist, in Gegenwart von suspendierten, Ruthenium enthaltenden Katalysatoren durchführt.

Dabei war überraschend, daß sich die Kondensation von Anilinen mit aliphatischen Ketonen und die Umwandlung der anfänglichen Kondensationsprodukte durch Variation der Molverhältnisse von Anilin:Keton bzw. von Anilin:Säure und in Abhängigkeit des Wassergehalts des Reaktionsgemisches sowie durch Anwendung eines höheren Drucks so steuern läßt, daß die gewünschten Diphenylmethanderivate mit hoher Selektivität erhalten werden.

Es war weiterhin unerwartet, daß diese Diphenylmethanderivate bei der Hydrierung in Gegenwart von speziellen Rutheniumkontakten in praktisch quantitativer Ausbeute in die entsprechenden alicyclischen Verbindungen übergeführt werden können.

Im folgenden sei das neue Verfahren genauer erläutert:

Neben dem Grundkörper lassen sich beim erfindungsgemäßen Verfahren auch an Kern und/oder Aminogruppe substituierte Aniline verwenden. Dabei kann der Kern durch eine oder zwei Alkylgruppen oder eine veresterte Carboxylgruppe in beliebiger Position, mit Ausnahme der para-Stellung substituiert sein, während sich die Substitution an der Aminogruppe auf einen Alkylrest beschränkt, z.B. o-Toluidin, m-Toluidin, N-Methylanilin, N-Ethylanilin, 2,6-Dimethylanilin, 2,6-Diethylanilin, 2,6-Diisopropylanilin, N-Methyl-o-toluidin, Anthranilsäureethylester.

Als aliphatische Ketone kommen neben Aceton solche Ketone in Betracht, bei denen der Ablauf der Kondensation nicht sterisch gehindert wird, beispielsweise 2-Butanon, 2-Pentanon, 3-Pentanon, 2-Hexanon, 3-Hexanon, Isobutylmethylketon.

Die zur Kondensation benötigten starken, ein- oder mehrbasigen Säuren sind insbesondere wasserlösliche Säuren mit einem $pK_A$-Wert kleiner 2,5, vorzugsweise kleiner 1,5. Als Beispiele sind zu nennen Salzsäure, Bromwasserstoffsäure, Schwefelsäure, Trifluoressigsäure, Methansulfonsäure, Trifluormethansulfonsäure, Benzolsulfonsäure, Phosphorsäure. Vorzugsweise wird Salzsäure verwendet.

Die Säuren können entweder in reiner Form oder zusammen mit ihren neutralen oder sauren Salzen, z.B. den Alkali- oder Ammoniumsalzen, eingesetzt werden. Beim erfindungsgemäßen Verfahren liegen sie in wäßriger Phase teilweise als Ammoniumsalze der zur Umsetzung benötigten Aniline vor.

Anstelle wasserlöslicher Säuren kommen auch wasserunlösliche, stark saure Ionenaustauscher in Betracht, deren $pK_A$-Werte im obengenannten Bereich liegen. Vorzugsweise werden Ionenaustauscherharze vom Typ der Copolymeren von hochsulfoniertem Styrol mit Divinylbenzol verwendet.

Beim Kondensationsschritt soll das Molverhältnis Anilin:Keton 2:1 bis 10:1, vorzugsweise 3:1 bis 6:1, betragen.

Es hat sich gezeigt, daß bei Werten kleiner 2:1 die Bildung unerwünschter Nebenprodukte gefördert wird.

Im Falle der Kondensation von unsubstituiertem Anilin mit Aceton treten dabei hauptsächlich folgende Nebenprodukte auf:
2,2,4-Trimethyl-1,2-dihydrochinolin, 2,3,4-Trimethylchinolin, 2,4-Dimethylchinolin, N-Isopropenyl-2,2-bis(4-aminophenyl)propan, N,N'-Diphenyl-2,2-bis(4-aminophenyl)propan.

Die Obergrenze mit dem Wert von 10:1 ist hinsichtlich der Entstehung von Nebenprodukten unerheblich. Allerdings werden bei weiterer Steigerung der Anilinkonzentration keine zusätzlichen Vorteile mehr erreicht.

Das Molverhältnis Anilin:Säureäquivalent kann zwischen 1:1 und 4:1, vorzugsweise 1:1 und 2:1, variiert werden. Die

Erhöhung über den Wert 4:1 hinaus hat in zunehmendem Maß die Bildung von Nebenprodukten zur Folge.

Bei der oben genannten Umsetzung von unsubstituiertem Anilin mit Aceton und Salzsäure handelt es sich dabei vor allem um Diphenylamin und dessen Folgeprodukte aus der Umsetzung mit 2,2-Bis(4-aminophenyl)propan. Andererseits wird bei einem Molverhältnis von Anilin:Säureäquivalent kleiner 1:1 die Bildungsgeschwindigkeit von 2,2-Bis-(4-aminophenyl)propan herabgesetzt, so daß Nebenreaktionen begünstigt werden, die im obigen Fall hauptsächlich zur Bildung von N-Isopropenylanilin und 2,2,4-Trimethyl--1,2-dihydrochinolin führen.

Beim Arbeiten mit sauren Ionenaustauschern setzt man jeweils die den wasserlöslichen Säuren äquivalente Mengen ein.

Die erfindungsgemäß zuzusetzende Menge Wasser liegt im Bereich von 10 bis 60 Gew.-%, bezogen auf das Reaktionsgemisch. Bei Wassermengen, die unter 10 Gew.-% liegen, wird die Bildung von Nebenprodukten begünstigt (im obigen Fall vor allem die Bildung von Chinolinen). Steigert man die Wassermenge über 60 Gew.-%, so wird das Verfahren unwirtschaftlich.

Die Reaktionskomponenten werden in einem Druckbehälter miteinander vereinigt. Dies geschieht zweckmäßigerweise durch Rühren, wobei die Reihenfolge der Zugabe der Reaktionspartner unerheblich ist. In einer bevorzugten Ausführungsform legt man das Amin vor und setzt dann Keton, Wasser und Säure zu.

Das Mischen der Reaktionspartner geschieht üblicherweise bei Raumtemperatur (20 bis 25°C). Anschließend wird die

Mischung dann unter Rühren im geschlossenen Reaktor auf eine Temperatur von 100 bis 160°C, vorzugsweise 120 bis 140°C, erhitzt. Die Temperaturerhöhung kann stufenweise oder kontinuierlich erfolgen, wobei es keine Rolle spielt, ob diesschnell oder langsam geschieht.

Die Kondensation wird üblicherweise bei einem Druck von 1 bis 20 bar durchgeführt. Im allgemeinen reicht dabei der Eigendruck des Systems aus. Er wird durch den Dampfdruck der leichtflüchtigen Reaktionsteilnehmer in Abhängigkeit von deren Temperatur und Konzentration bestimmt. Der Druck kann auch mit Hilfe eines Inertgases, beispielsweise Stickstoff, erhöht werden, jedoch bringt eine Erhöhung über 20 bar keine Verbesserung hinsichtlich Umsatz und Selektivität.

Die Reaktionszeit beträgt im allgemeinen 2 bis 20 Stunden. Der Fortschritt der Reaktion kann verfolgt werden, wenn man aus dem Druckgefäß beispielsweise über ein Tauchrohr aliquote Teile des Reaktionsgemisches entnimmt, diese mit Natronlauge (25 Gew.-%) schwach alkalisch stellt, in die wäßrige und organische Phase trennt und die organische Phase gas- oder flüssigkeitschromatographisch analysiert.

Die Umsetzung läßt sich sowohl diskontinuierlich wie auch kontinuierlich durchführen. Bei kontinuierlicher Reaktionsführung ist es vorteilhaft, in einer Rührkesselkaskade mit zwei oder mehr entsprechend ausgerüsteten Rührkesseln zu arbeiten.

Das erfindungsgemäß erhaltene Reaktionsgemisch ist, abhängig vom verwendeten sauren Katalysator, entweder heterogen (fest-flüssig) oder homogen (flüssig) und läßt sich auf verschiedene Weise weiter- bzw. nachbehandeln. In der Regel wird der Reaktionsaustrag mit Natronlauge von 25 bis

40 Gew.-% auf einen pH-Wert von 8 bis 10 gestellt, einige Zeit bei 40°C gerührt und anschließend in die wäßrige und organische Phase getrennt.

In der wäßrigen Phase partiell gelöste Ausgangsstoffe, beispielsweise Aceton und Anilin, können durch Extraktion mit einem hydrophoben Lösungsmittel weitgehend zurückgewonnen werden. Als geeignete hydrophobe Lösungsmittel kommen beliebige mit Wasser nicht mischbare, gegenüber den Reaktionskomponenten inerte Lösungsmittel wie Chlorbenzol, Benzol, Toluol, Xylole, Dichlorethan, Chloroform, Tetrachlorkohlenstoff u.ä. in Betracht. Besonders bevorzugtes Lösungsmittel ist o-Xylol.

Bei der Verwendung fester hochsulfonierter, mit Divinylbenzol vernetzter Polystyrol-Ionenaustauscherharze wird nach Zugabe der Natronlauge der feste Katalysator durch Filtration abgetrennt.

Die mit Wasser gewaschene organische Phase und die vereinigten Extrakte werden, getrennt oder vereinigt, zuerst unter Normaldruck und dann unter reduziertem Druck fraktioniert destilliert. Die dabei erhaltenen Diphenylmethanderivate werden in den Hydrierprozeß eingesetzt, während zurückgewonnene Einsatzstoffe wieder in die Kondensationsstufe rückgeführt werden können.

Die Hydrierung wird bei einem Druck, der größer als 50 bar ist, durchgeführt. Im allgemeinen wird ein Druck von 60 bis 300 bar, vorzugsweise 150 bis 300 bar, angewendet.

Zweckmäßig wird für das Verfahren eine Temperatur zwischen 30 und 250°C gewählt, wobei der Bereich von 120 bis 180°C besonders bevorzugt ist.

Die zu hydrierenden Produkte können sowohl in Substanz als auch in Lösung eingesetzt werden. Es erweist sich aber als vorteilhaft, die Hydrierung in Lösung durchzuführen, wobei als Lösungsmittel Ether verwendet werden können, z.B. Diethylether, Glykoldimethylether, Dioxan, Tetrahydrofuran.

Geeignete, Ruthenium enthaltende Katalysatoren sind solche, die in situ, d.h. im Reaktionsgemisch durch Reduktion von Ruthenium-(IV)-oxidhydrat mit Wasserstoff erhalten werden. Diese Katalysatorvorstufe kann man entweder in suspendierter Form oder als getrocknetes Pulver oder aufgebracht auf übliche Trägermaterialien, wie Kieselsäure, Aluminiumoxid oder Aktivkohle, gewinnen. Die Herstellung von Ruthenium-(IV)-oxidhydrat aus Rutheniumtrichloridtrihydrat, $RuCl_3 \cdot 3H_2O$ ist in der DE-PS 2 132 547 näher beschrieben.

Zu einem besonders aktiven Hydrierkatalysator gelangt man, wenn man eine pulverförmige Katalysatorvorstufe mit einem Gehalt von ca. 60 bis 70 Gew.-% Ruthenium verwendet, deren Teilchengröße im Bereich von 40 bis 60 nm liegt.

Es ist möglich, die Katalysatorvorstufe entweder feucht einzusetzen oder sie vor dem Gebrauch einer Trocknung zu unterziehen. Dies läßt sich durch Erhitzen im Vakuum auf eine Temperatur von 80 bis 100°C erreichen. Man kann aber auch die zentrifugenfeuchte Katalysatorvorstufe mit einem organischen Lösungsmittel wasserfrei waschen und in Form eines suspendierten Konzentrats, am besten in dem für die Hydrierung vorgesehenen Lösungsmittel, einsetzen.

Im allgemeinen genügen Mengen von 10 bis 1000 ppm Ruthenium-(IV)-oxidhydrat, vorzugsweise von 10 bis 100 ppm, bezogen auf das Diphenylmethanderivat.

Die folgenden Beispiele sollen das erfindungsgemäße Verfahren näher veranschaulichen. Die dort genannten Teile bedeuten Gewichtsteile.

Beispiel 1

a) Ein emaillierter Autoklav, ausgerüstet mit Doppelmantelheizung und Thermoölkreis, Rührer, Thermometer, Zugabevorrichtung, Berstscheibe (40 bar) und Tauchrohr mit Probenahme, wurde mit 521 Teilen Anilin, 116 Teilen Aceton, 200 Teilen Wasser und 568 Teilen Salzsäure von 36 Gew.-% beschickt, geschlossen und unter Rühren im Verlaufe von ca. 1 Std. auf 130°C erhitzt, wobei sich ein Druck von 3,2 bar einstellte.

Von Zeit zu Zeit wurde über das Tauchrohr eine Probe entnommen, von der nach Neutralisation mit Natronlauge die organische Phase abgetrennt, destilliert und gaschromatographisch analysiert wurde. Nach Ablauf von 15 Std. wurde das Reaktionsgemisch in einen Glaskolben überführt, mit 565 Teilen 40%iger Natronlauge versetzt, kurze Zeit bei 30 bis 40°C gerührt und anschließend in einem Scheidetrichter in wäßrige und organische Phase getrennt.

Die unter den genannten Bedingungen mit Anilin und Aceton gesättigte wäßrige Phase extrahierte man zweimal mit je 100 Teilen o-Xylol und vereinigte die anfallende Extraktphase mit der organischen Phase des Reaktionsaustrags. Die vereinigten organischen Phasen wurden mehrfach mit Wasser chloridfrei gewaschen und anschließend in einer zweistufigen Destillationsapparatur aufgearbeitet.

In der ersten Stufe destillierte man Aceton, Anilin, o-Xylol und N-Isopropenylanilin unter Normaldruck ab; die Kondensationsprodukte befanden sich dabei im Destillationssumpf. Man gewann 23 Teile Aceton und 233 Teile Anilin entsprechend einem Umsatz von 77,5 %

bezogen auf Anilin und 79,8 % bezogen auf Aceton zurück. Der Destillationssumpf wurde in der zweiten Stufe mit Hilfe einer mit Drahtnetzkörpern gefüllten, mindestens 15 Böden aufweisenden Kolonne bei 0,5 mbar fraktioniert destilliert. Man erhielt 292 Teile 2,2-Bis(4-aminophenyl)propan in einer Reinheit von 97 % (Sdp. 183 bis 184°C bei 0,5 mbar, Fp: 132°C) entsprechend einer Selektivität von 82 %.

b) 170 Teile des gemäß Beispiel 1a) erhaltenen 2,2-Bis-(4-aminophenyl)propans, 250 Teile Dioxan und 0,04 Teile eines bei 100°C im Vakuum von 30 mbar getrockneten Ruthenium-(IV)-oxidhydrats wurden in einem Rollautoklaven nach Spülung mit Stickstoff und Füllung mit Wasserstoff auf 150°C erhitzt und der Wasserstoffdruck auf 250 bar eingestellt. Man hydrierte, bis kein Wasserstoff mehr aufgenommen wurde. Danach trennte man den Katalysator vom Reaktionsaustrag ab, entfernte das Lösungsmittel unter reduziertem Druck und fraktionierte den Rückstand bei 4 mbar. Man erhielt 171 Teile 2,2-Bis(4-aminocyclohexyl)propan (Sdp: 168 bis 170°C) entsprechend einer Ausbeute von 95 %.

Beispiel 2

a) In der in Beispiel 1a) beschriebenen Druckapparatur wurden 372 Teile Anilin, 116 Teile Aceton, 500 Teile Wasser und 800 Teile eines sulfonierten mit Dinvinylbenzol vernetzten Polystyrolharzes mit folgenden Kenndaten eingesetzt:

| | |
|---|---|
| Korngröße: | 0,3 - 1,5 mm |
| Wassergehalt: | 50 - 55 % |

$H^+$- Totalkapazität: 4,9 Milliäquivalent $H^+$/g

Das Gemisch wurde verschlossen und unter Rühren 20 Std. lang auf 130°C erhitzt, wobei sich ein Druck von 3,0 bar einstellte. Den sauren Katalysator entfernte man anschließend durch Filtration. Der flüssige Reaktionsaustrag wurde in die wäßrige und organische Phase aufgetrennt und analog Beispiel 1a) aufgearbeitet. Man gewann 25 Teile Aceton und 86 Teile Anilin entsprechend einem Umsatz von 77 % bezogen auf Anilin und 78,5 % bezogen auf Aceton zurück und erhielt 350 Teile 2,2-Bis(4-aminophenyl)propan mit einer Reinheit von 95 % entsprechend einer Selektivität von 77 %.

b) Einen Schüttelautoklaven beschickte man mit 300 Teilen Dioxan, 200 Teilen des gemäß Beispiel 3a erhaltenen 2,2-Bis(4-aminophenyl)propans und 0,5 Teilen eines zentrifugenfeuchten, mit Dioxan gewaschenen Ruthenium-(IV)-oxidhydrats (19 Gew.-% Ru). Das Reaktionsgemisch wurde bei einem Druck von 300 bar und einer Temperatur von 150°C hydriert und analog Beispiel 1b) aufgearbeitet. Man erhielt 190 Teile 2,2-Bis(4-aminocyclohexyl)propan entsprechend einer Ausbeute von 95 %.

Beispiel 3

a) In der in Beispiel 1a) beschriebenen Druckapparatur wurden 600 Teile o-Toluidin, 116 Teile Aceton, 200 Teile Wasser und 568 Teile Salzsäure von 36 Gew.-% 20 Std. lang auf 130°C erhitzt, wobei sich ein Druck von 3,1 bar einstellte. Das Reaktionsgemisch wurde in einen Glaskolben überführt, mit 565 Teilen Natronlauge von 40 Gew.-% versetzt, kurze Zeit bei 30°C gerührt und anschließend in einem Scheidetrichter in die wäßrige und organische Phase

getrennt. Die organische Phase wurde mehrfach mit Natronlauge von 10 Gew.-% chloridfrei gewaschen und destillativ aufgearbeitet.

In der ersten Stufe wurden Aceton, o-Toluidin und N-Isopropenyl-o-toluidin bei 140 mbar abdestilliert und die Kondensationsprodukte als Destillationssumpf erhalten. Dabei gewann man 301 Teile o-Toluidin und 34 Teile Aceton entsprechend einem Umsatz von 69,7 % bezogen auf o-Toluidin und 70,7 % bezogen auf Aceton zurück. Der Destillationssumpf wurde analog Beispiel 1a) fraktioniert destilliert, dabei erhielt man 336 Teile 2,2-Bis(4-amino-3-methylphenyl)propan (Selektivität: 63 %, Sdp. 197 - 200°C bei 0,5 mbar), 6 Teile 2,2,4,8-Tetramethyl-1,2-dihydrochinolin und 8 Teile N-Isopropenyl-2,2-bis(4-amino-3-methylphenyl) propan.

b) Man beschickte einen Schüttelautoklaven mit 200 Teilen des gemäß Beispiel 3a) erhaltenen 2,2-Bis(4-amino-3-methylphenyl)propans, 300 Teilen Dioxan und 0,1 Teilen eines bei 100°C im Vakuum von 30 mbar getrockneten Ruthenium-(IV)-oxidhydrats (67 Gew.-% Ru). Das Reaktionsgemisch wurde bei einem Druck von 200 bar und bei 200°C hydriert und analog Beispiel 1b) aufgearbeitet. Man erhielt 197 Teile 2,2-Bis(4-amino-3-methylcyclohexyl)propan (Sdp. 190 bis 193°C bei 4 mbar) entsprechend einer Ausbeute von 94 %.

Beispiel 4

a) In der in Beispiel 1a) beschriebenen Druckapparatur wurden 521 Teile Anilin, 144 Teile Methylethylketon, 200 Teile Wasser und 568 Teile Salzsäure von

36 Gew.-% 20 Std. lang auf 130°C erhitzt, wobei sich ein Druck von 3,2 bar einstellte. Das Reaktionsprodukt wurde analog Beispiel 3a) bis zur zweistufigen Destillation aufgearbeitet.

In der ersten Stufe wurden Methylethylketon, Anilin und N-Isobutenylanilin bei 140 mbar abdestilliert und die Kondensationsprodukte als Destillationssumpf erhalten.

Dabei wurden 29 Teile Methylethylketon und 225 Teile Anilin entsprechend einem Umsatz von 79,5 % bezogen auf Anilin und 80 % bezogen auf Methylethylketon zurückgewonnen. Der Destillationssumpf wurde analog Beispiel 1a) fraktioniert destilliert, wobei man 362 Teile 2,2-Bis(4-aminophenyl)butan (Sdp. 186 bis 188°C bei 0,5 mbar) entsprechend einer Selektivität von 75,2 % erhielt.

b) Man beschickte einen Schüttelautoklaven mit 200 Teilen des gemäß Beispiel 4a) erhaltenen 2,2-Bis(4-aminophenyl)butans, 300 Teilen Dioxan und 0,1 Teilen eines bei 100°C im Vakuum von 30 mbar getrockneten Ruthenium-(IV)-oxidhydrats (67 Gew.-% Ru). Das Reaktionsgemisch wurde bei einem Druck von 200 bar und bei 200°C hydriert und analog Beispiel 1b) aufgearbeitet. Man erhielt 196 Teile 2,2-Bis(4-aminocyclohexyl)butan (Sdp. 173 bis 175°C bei 4 mbar) entsprechend einer Ausbeute von 93 %.

Vergleichsbeispiel 1

Unter Benutzung der in Beispiel 1a) beschriebenen Druckapparatur wurden 559 Teile Anilin, 232 Teile Aceton, 410 Teile Wasser und 203 Teile Salzsäure von 36 Gew.-%

20 Std. lang auf 130°C erhitzt, wobei sich ein Druck von 6 bar einstellte. Nach Zugabe von 202 Teilen Natronlauge von 40 Gew.-%, Phasentrennung und zweistufiger Destillation der organischen Phase wurden 28 Teile Aceton und 271 Teile Anilin entsprechend einem Umsatz von 38,7 % bezogen auf Anilin zurückgewonnen und als Kondensationsprodukte 70 Teile 2,2,4-Trimethyl-1,2-dihydrochinolin (Sdp. 130°C, 10 mbar, Fp. 21°C), 28 Teile N-Isopropenyl-2,2-bis(4-aminophenyl)propan und 71 Teile 2,2-Bis-(4-aminophenyl)propan entsprechend einer Selektivität von 20,3 % erhalten. 150 Teile waren Destillationsrückstand.

Vergleichsbeispiel 2

In der in Beispiel 1a) beschriebenen Druckapparatur wurden 521 Teile Anilin, 116 Teile Aceton und 142 Teile Salzsäure von 36 Gew.-% 20 Std. lang auf 130°C erhitzt, wobei sich ein Druck von 3,5 bar einstellte. Das Reaktionsprodukt wurde in einen Glaskolben überführt, mit 145 Teilen Natronlauge von 40 Gew.-%, versetzt, kurze Zeit bei 40°C gerührt und in die organische und wäßrige Phase getrennt. Nach zweistufiger Destillation wurden 21 Teile Aceton und 309 Teile Anilin entsprechend einem Umsatz von 56,8 % bezogen auf Anilin zurückgewonnen und als Kondensationsprodukte 31 Teile 2,2,4-Trimethyl-1,2-dihydrochinolin, 80 Teile N-Isopropenyl-2,2-bis(4-aminophenyl)propan, 12 Teile N,N'-Diphenyl-2,2-bis(4-aminophenyl)propan und 52 Teile 2,2-Bis(4-aminophenyl)propan entsprechend einer Selektivität von 20,2 % erhalten. 127 Teile waren Destillationsrückstand.

Patentansprüche

1. Verfahren zur Herstellung von Bis(aminocyclohexyl)-dialkylmethanen der allgemeinen Formel I

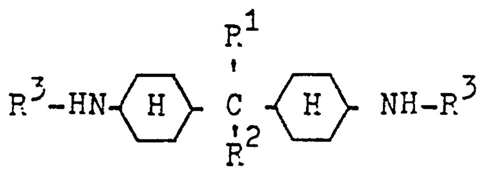 (I)

in der
$R^1$ und $R^2$ gleich oder verschieden sein können und eine niedere Alkylgruppe mit 1 bis 4 Kohlenstoffatomen und
$R^3$ ein Wasserstoffatom oder eine niedere Alkylgruppe mit 1 bis 4 Kohlenstoffatomen bedeuten, wobei die beiden Cyclohexanringe gegebenenfalls jeweils durch eine oder zwei niedere Alkylgruppen, mit 1 bis 4 Kohlenstoffatomen oder durch eine Carboxyalkylgruppe mit 1 bis 4 Kohlenstoffatomen im Alkylrest substituiert sein können,
durch Kondensation von in para-Stellung unsubstituierten Anilinen der allgemeinen Formel II

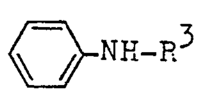 (II)

in der $R^3$ die vorgenannte Bedeutung besitzt und die gegebenenfalls am Kern durch eine oder zwei niedere Alkylgruppen, mit 1 bis 4 Kohlenstoffatomen oder durch eine Carboxyalkylgruppe mit 1 bis 4 Kohlenstoffatomen im Alkylrest substituiert sein können, mit niederen aliphatischen Ketonen, deren Alkylreste jeweils 1 bis 4 Kohlenstoffatome aufweisen, in Gegenwart einer starken, ein- oder mehrbasigen Säure und nachfolgende Hydrierung der so erhaltenen Produkte, dadurch gekennzeichnet, daß man die Kondensation bei einer Temperatur von 100 bis 160°C und einem Druck von 1 bis 20 bar, in Anwesenheit von 10 bis 60 Gew.-% Wasser, bezogen auf das Reaktionsgemisch, durchführt, wobei das Molverhältnis Anilin:Keton 2:1 bis 10:1 und das Molverhältnis Anilin:Säureäquivalent 1:1 bis 4:1 beträgt, und die nachfolgende Hydrierung der so erhaltenen Produkte bei einer Temperatur von 30 bis 250°C und einem Druck, der größer als 50 bar ist, in Gegenwart von suspendierten, Ruthenium enthaltenden Katalysatoren durchführt.

2. Verfahren gemäß Anspruch 1, dadurch gekennzeichnet, daß man als Säure Salzsäure verwendet.

3. Verfahren gemäß Anspruch 1, dadurch gekennzeichnet, daß man als Säure einen wasserunlöslichen, stark sauren Ionenaustauscher vom Typ hochsulfonierter mit Divinylbenzol vernetzter Polystyrolharze verwendet.

4. Verfahren gemäß Anspruch 1, dadurch gekennzeichnet, daß man die Kondensation bei einer Temperatur von 120 bis 140°C durchführt.

5. Verfahren gemäß Anspruch 1, dadurch gekennzeichnet, daß das Molverhältnis Anilin:Keton 3:1 bis 6:1 beträgt.

6. Verfahren gemäß Anspruch 1, dadurch gekennzeichnet, daß das Molverhältnis Anilin:Säureäquivalent 1:1 bis 2:1 beträgt.

7. Verfahren gemäß Anspruch 1, dadurch gekennzeichnet, daß man die Hydrierung bei einer Temperatur von 120 bis 180°C durchführt.

8. Verfahren gemäß Anspruch 1, dadurch gekennzeichnet, daß man die Hydrierung bei einem Druck von 150 bis 300 bar durchführt.

0099516

Europäisches Patentamt

# EUROPÄISCHER RECHERCHENBERICHT

Nummer der Anmeldung

EP 83 10 6607

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-1 793 020 (SOC. RHODIACETA) * Insgesamt * | 1-8 | C 07 C 85/24 |
| X | LU-A- 57 274 (SOC. RHODIACETA) * Beispiele * | 1-8 | |
| X | DIE MAKROMOLEKULARE CHEMIE, Band XXXII, 1959, Seiten 1-12 N. YODA: "Synthesis of polyanhydrides. II. New aromatic polyanhydrides with high melting points and fiber-forming properties" * Seite 6 * | 1-8 | |
| X | CHEMICAL ABSTRACTS, Band 87, Nr. 1, 4. Juli 1977, Seite 442, Nr. 5500u, Columbus, Ohio, USA & JP - A - 76 125 363 (UBE INDUSTRIES, LTD.) 01-11-1976 * Insgesamt * | 1-8 | RECHERCHIERTE SACHGEBIETE (Int. Cl. 3) |
| | | | C 07 C 85/00 |
| X | FR-A-2 298 529 (BAYER A.G.) * Seite 17, Tafel * | 1-8 | |
| Y | DE-A-2 945 614 (BAYER A.G.) * Ansprüche * | 1-8 | |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 25-10-1983 | PAUWELS G.R.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

0099516

Europäisches Patentamt

# EUROPÄISCHER RECHERCHENBERICHT

Nummer der Anmeldung

EP 83 10 6607

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | US-A-2 606 925 (G.M. WHITMAN) * Beispiele 5-12; Spalte 6, Zeile 60 - Spalte 7, Zeile 3 * --- | 1-8 | |
| Y | DE-A-2 037 550 (KAISER ALUMINUM & CHEMICAL CORP.) * Seiten 5-10 * ----- | 1-8 | |
| | | | RECHERCHIERTE SACHGEBIETE (Int. Cl. 3) |

Der vorliegende Recherchenbericht wurde für alle Patentansprüche erstellt.

| Recherchenort | Abschlußdatum der Recherche | Prüfer |
|---|---|---|
| DEN HAAG | 25-10-1983 | PAUWELS G.R.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82